# EUROPEAN PATENT SPECIFICATION

(11) **EP 3 617 330 B1**
(45) Date of publication and mention of the grant of the patent: **27.12.2023**
(21) Application number: 19203410.6
(22) Date of filing: 30.03.2012
(51) Int. Cl.: C12Q 1/6886, A61K 31/517, A61K 31/58

(54) **BIOMARKERS FOR PREDICTING SENSITIVITY TO CANCER TREATMENTS**
BIOMARKER ZUR VORHERSAGE DES ANSPRECHENS AUF KREBSBEHANDLUNGEN
BIOMARQUEURS POUR PRÉDIRE LA SENSIBILITÉ AUX TRAITEMENTS DU CANCER

(30) Priority: 01.04.2011 US 201161471036 P
(43) Date of publication of application: 04.03.2020
(62) Divisional of application: 12765178.4
(73) Proprietor: GENENTECH, INC., South San Francisco, CA 94080-4990 (US)
(72) Inventor: LIN, Kui, South San Francisco, CA California 94080-4990 (US); PUNNOOSE, Elizabeth, South San Francisco, CA California 94080-4990 (US); SESHAGIRI, Somasekar, South San Francisco, CA California 94080-4990 (US)
(74) Representative: Mewburn Ellis LLP

(56) References cited:
- WO-A2-2008/049022
- Josep Tabernero ET AL: "Targeting the PI3K-Akt-mTOR pathway with GDC-0068, a novel selective ATP competitive Akt inhibitor", , 9 March 2011 (2011-03-09), XP055153957, Retrieved from the Internet: URL:http://www.arraybiopharma.com/files/20 13/9810/8011/PubAttachment437.pdf [retrieved on 2014-11-19]
- LIN K ET AL: "79 Preclinical characterization of GDC-0068, a novel selective ATP competitive inhibitor of Akt", EUROPEAN JOURNAL OF CANCER. SUPPLEMENT, PERGAMON, OXFORD, GB, vol. 8, no. 7, 1 November 2010 (2010-11-01), page 33, XP027497767, ISSN: 1359-6349, DOI: 10.1016/S1359-6349(10)71784-5 [retrieved on 2010-11-01]
- MARGRITH E MATTMANN ET AL: "Inhibition of Akt with small molecules and biologics: historical perspective and current status of the patent landscape/x-ms-", EXPERT OPINION ON THERAPEUTIC PATENTS,, vol. 21, no. 9, 1 January 2011 (2011-01-01), pages 1309-1338, XP008159752, ISSN: 1354-3776, DOI: 10.1517/13543776.2011.587959
- CARPTEN JOHN D ET AL: "A transforming mutation in the pleckstrin homology domain of AKT1 in cancer", NATURE, MACMILLAN JOURNALS LTD, LONDON, vol. 448, no. 7152, 1 July 2007 (2007-07-01), pages 439-445, XP002484330, ISSN: 0028-0836, DOI: 10.1038/NATURE05933
- ENGELMAN JEFFREY A: "Targeting PI3K signalling in cancer: opportunities, challenges and limitations", NATURE REVIEWS. CANCER, NATUR PUBLISHING GROUP, LONDON, GB, vol. 9, no. 8, 1 August 2009 (2009-08-01), pages 550-562, XP002693047, ISSN: 1474-175X, DOI: 10.1038/NRC.2664

## Description

### Background

Cancer can arise when cells have mutations that ultimately confer a growth advantage to the cells. Somatic mutations include, *e.g*., nucleotide base substitutions, deletions, insertions, amplifications, and rearrangements. Identification of somatic mutations that occur in cancer provides valuable information regarding the development of cancer. Such information is also useful for the identification of diagnostic markers and therapeutic targets in cancer. (*see, e.g.,* Bamford et al. (2004) British. Journal of Cancer 91:355-358.) The identification of somatic mutations associated with cancer has proven valuable in clinical settings, *e.g*., in distinguishing patient populations that would be responsive to a particular therapy. (*see, e.g.,* Lynch et al. (2004) N. Engl. J. Med. 350:2129-2139; O'Hare (2004) Blood 104:2532-2539.) Thus, a continuing need exists to identify somatic mutations that occur in cancer.

Germline variations, or polymorphisms, are heritable variations that are present in an organism's genome. Polymorphisms include restriction fragment length polymorphisms (RFLPs), short tandem repeats (STRs), and single nucleotide polymorphisms (SNPs). Germline variations can also be associated with susceptibility to certain diseases, including cancer. (*see, e.g.,* Vierimaa et al. (2006) Science 312:1228-1230; Landi et al. (2006) Science 313:521-522; Zhu et al. (2004) Cancer Research 64:2251-2257.) Thus, a continuing need exists to identify polymorphisms associated with cancer.
Josep Tabemero et al., "Targeting the PI3K-Akt-mTOR pathway with GDC-0068, a novel selective ATP competitive Akt inhibitor" - URL: http:// www.arraybiopharma.com/files/2013/9810/8011/PubAttachment437.pdf is a presentation indicating that GDC-0068 is an oral selective ATP-competitive AKT inihibitor.
WO 2008/049022 describes a single nucleotide polymorphism in the AKT1 gene that can be used to determine if a subject can be treated (or is resistant to treatment with) a chemotherapeutic agent.

### Summary of Certain Embodiments of the Invention

The inventions described herein meet the above-described needs and provide other benefits.

Applicants have discovered that biomarkers can predict the efficacy of AKT inhibitors in treating hyperproliferative disorders, such as cancer.

Applicants have discovered that certain mutations in *AKT* can lead to disrupted interactions between the AKT PH domain and kinase domain. A disruption between these domains, caused by the mutation(s), appears to lead to constitutive phosphorylation of AKT and to constitutive AKT signaling. These effects also allow for the transformation of cells. These mutations confer resistance to PI3K and allosteric Akt inhibitors. Accordingly, the presence of such mutations indicate that the effective dosage for PI3K and allosteric Akt inhibitors will be higher, and also indicates that inhibitors other than PI3K and/or allosteric Akt inhibitors should be used, such as ATP-competitive Akt inhibitors. The use of this biomarker, either alone or in combination with other biomarkers described herein, is useful for predicting the sensitivity of the growth of a tumor cell to an AKT inhibitor, administered either alone, or in combination with another therapeutic compound, such as 5-FU, a platinum agent (carboplatin, cisplatnin, oxaliplatin, etc.) irinotecan, docetaxel, doxorubicin, gemcitabine, SN-38, capecitabine, temozolomide, erlotinib, PD-0325901, paclitaxel, bevacizumab, pertuzumab, tamoxifen, rapamycin, lapatinib, PLX-4032, MDV3100, abiraterone, and GDC-0973 and other MEK inhibitors.
The present invention comprises a method of predicting the sensitivity of the growth of a tumour cell to an AKT inhibitor according to claim 1. The inventicon also provides GDC-0068 or a salt thereof for use in a method of treating a cancer cell according to claim 15.

### Brief Description of the Figures

Figure 1 depicts AKT and the interactions of the PH and kinase domain (1A and 1B) and also depicts the locations of interactions between those domains (1C).
Figure 2 depicts results indicating that synthetic mutations at sites thought to disrupt the interactions of PH domain with the kinase domain lead to constitutive phosphorylation of Akt.
Figure 3 depicts somatic mutations.
Figure 4 depicts results indicating that somatic mutations, which lead to constitutive Akt phosphorylation, lead to constitutive Akt signaling.
Figure 5 depicts results indicating that Akt1 mutants can transform cells.
Figure 6 depicts results indicating that Akt1 mutants confer resistance to PI3K inhibitors and to AKT allosteric inhibitors.
Figure 7 depicts changes in pPRAS40 T246 in tumors from patients treated with GDC-0068.

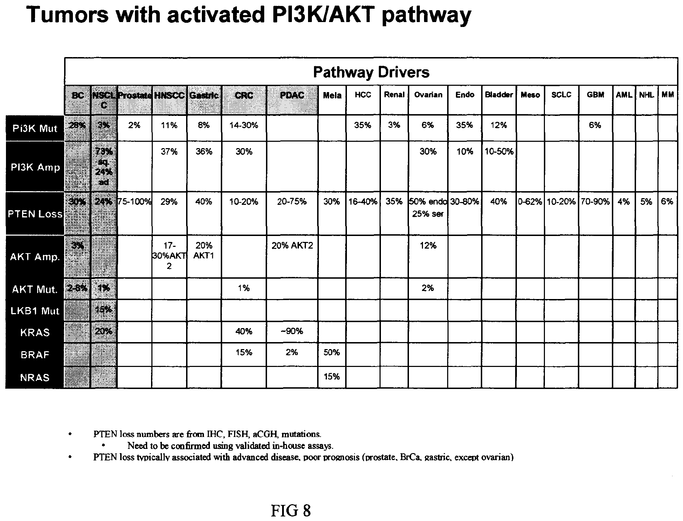

Figure 8 depicts tumors with an activated PI3K/AKT pathway.
Figure 9 depicts results indicating that a high AKT activity profile predicts sensitivity to GDC-0068.

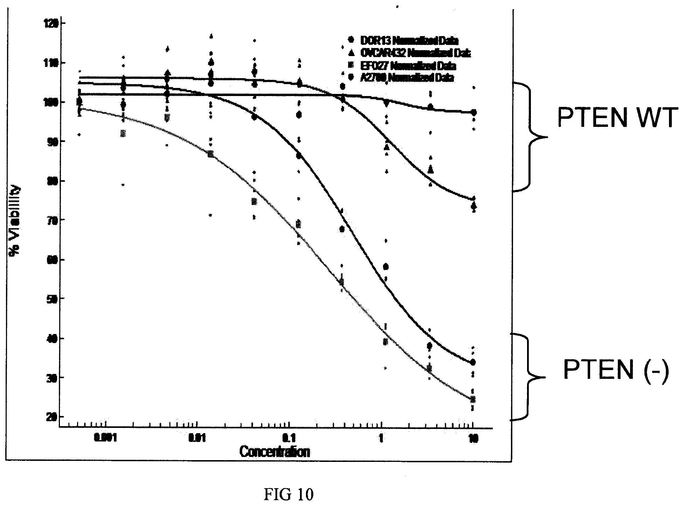

Figure 10 depicts results demonstrating a strong correlation with PTEN loss and sensitivity to GDC-0068 in prostate and ovarian cell lines. The results are of a normalized single compound dose response in GDC-0068.

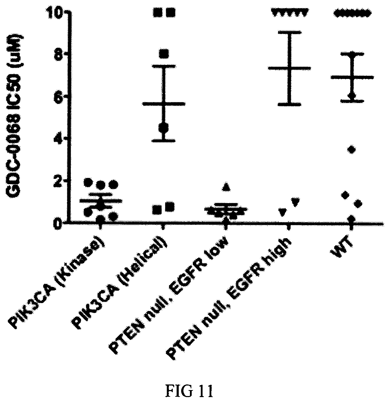

Figure 11 depicts results demonstrating that PTEN loss and PIK3CA mutations are strongly correlated with GDC-0068 single agent sensitivity *in vitro.*

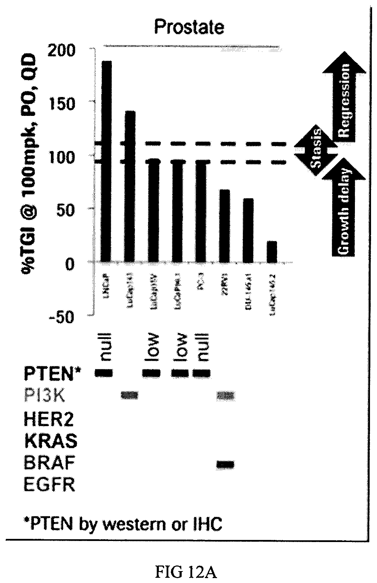

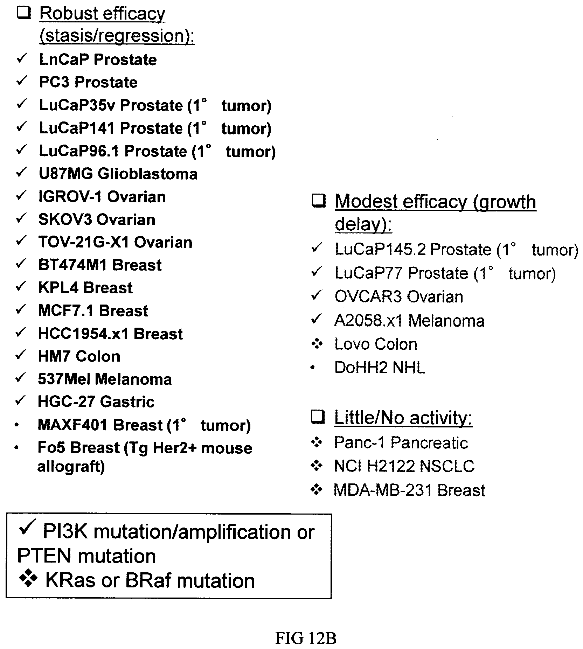

Figure 12A and 12B depicts results of GDC-0068 single agent activity in xenograft models (12A) and in vitro cell line screening data (12B) indicating that the highest percentage of tumor growth inhibition also have evidence of pathway activation either through loss of PTEN or PI3K mutation. Robust efficacy is observed in models with AKT pathway activation (and without MEK Pathway Activation).

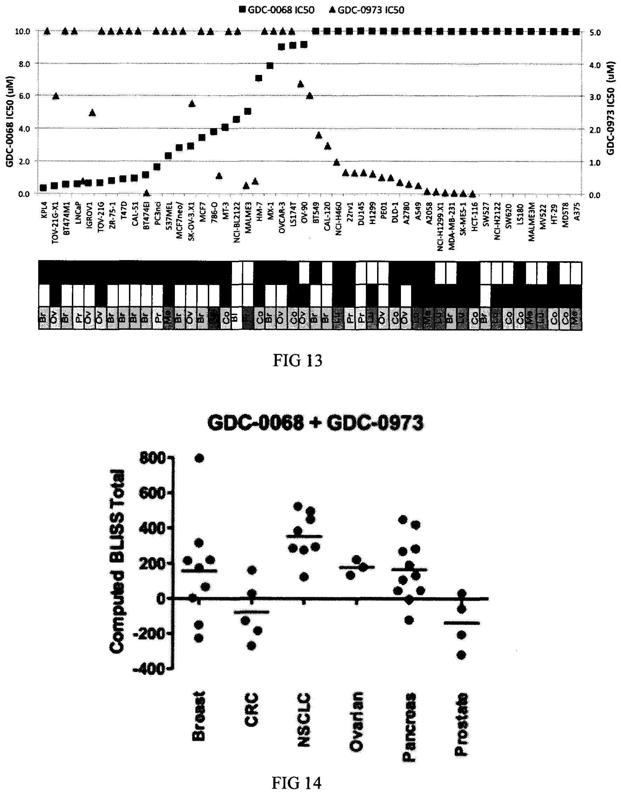

Figure 13 depicts results indicating a negative correlation between GDC-0068 and MEK inhibitor single agent sensitivity of a variety of cell lines.
Figure 14 depicts results demonstrating a strong synergy in cell lines with AKT pathway activation (PTEN loss, PI3K mutations). BLISS analysis indicates broad synergy for GDC-0068 in combination with a MEK inhibitor (GDC-0973).

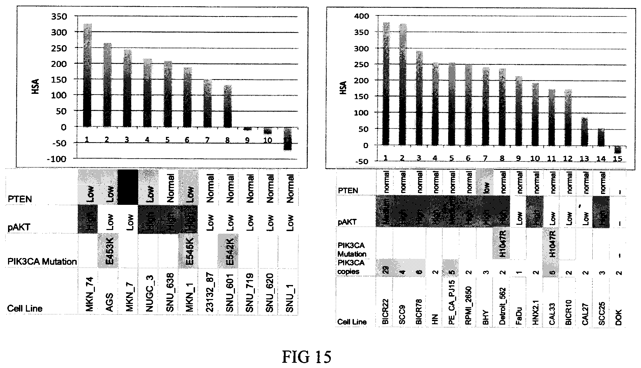

Figure 15 depicts results that the combination effects of GDC-0068 with Cisplatin +5FU are associated with AKT pathway activation. Combo screens with 5FU/Cisplatin (FOLFOX) show evidence of additive effects. Additive effects are associated with pathway activation: PTEN, pAKT, PI3K mutation.

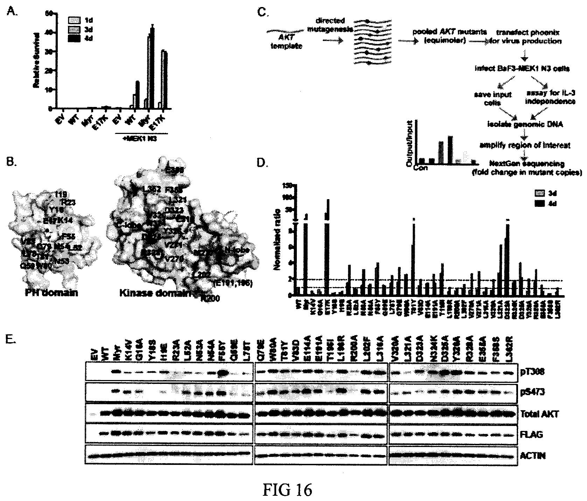

Figure 16 PH-kinase domain contact site mutations lead to AKT activation. (A) IL-3 independent proliferation of BaF3 cells stably expressing empty vector (EV), wild type (WT), myristoylated (Myr) or E17K AKT1 alone or in combination with MEK1 N3. (B) An "open book" representation of PH and KD of AKT1 in complex with an allosteric inhibitor (PDB Accession Code 3O96). (C) Schematic depicting the screen used to assess the effect of AKT1 PH-KD interface mutations. (D) PH-KD interface mutations promote IL-3 independent proliferation of BaF3 cells. (E) Immunoblot analysis of NIH3T3 cells stably expressing empty vector and the indicated AKT1 constructs.
Figure 17. Somatic *AKT* mutations in human cancer. Somatic mutations in AKT family members. Horizontal black bars indicate residues conserved across AKT 1, 2 and 3.

### Detailed Description

Recent structural studies indicate that inhibitory inter-domain interactions play a crucial role in regulating AKT activation. Using a mutational screen, it is show here that activation of AKT can result from mutations in residues involved in PH-kinase domain contacts. Further, the identification of novel mutations in human cancers are reported, some of which involve residues at the PH-KD interface.

In addition to the previously identified mutation E17K, the AKT1 PH domain mutant L52R and the kinase domain mutant D323H identified in clinical samples mediate cellular transformation and are oncogenic *in vivo.* Inspection of the structure of full length AKT1 reveals that E17, L52 and D323 lie at the PH-KD interface and substitutions at these positions are predicted to perturb PH-KD binding. Consistent with this, both L52R and D323H weaken PH-KD binding in 2-hybrid assays. Previously the mechanism of activation of E17K has been attributed to an altered lipid-binding specificity. These results indicate perturbation of inter-domain interactions to be an additional mechanism underlying E17K activation. Taken together these findings suggest that the oncogenicity of the *AKT1* PH-KD interface mutations identified here stems from destabilization of inter-domain contacts.

Inhibitors targeting the PI3K-AKT pathway members including AKT are currently in various stages of development. Previous studies have shown that AKT allosteric inhibitors require an intact PH-KD interface as such inhibitors preferentially bind the closed "PH-in" conformation. Consistent with this, mutations in *AKT* that favor an open ("PH-out") conformation show reduced sensitivity to allosteric AKT inhibitors, although they retain sensitivity to ATP-competitive inhibitors. This indicates that the *AKT* mutational status has important implications for the choice of inhibitor in the clinic. *AKT* mutations, while may function as drivers in naive tumors, can also arise in tumors in response to agents that target upstream components of the AKT pathway.

In certain embodiments, the presence of B-Raf or K-Ras mutations are negative predictors (*i.e.,* contraindicated) and those patients should be selected out from the treatment group to receive AKT inhibitors, such as GDC-0068.

GDC-0068, and similar ATP competitive inhibitors, preferentially target active Akt and lock Akt in a hyperphosphorylated but inactive state by blocking dephosphorylation. An increase in pAkt can be used as a pharmacodynamic biomarker ("PD biomarkers") for the effects of GDC-0068 and similar ATP competitive inhibitors.

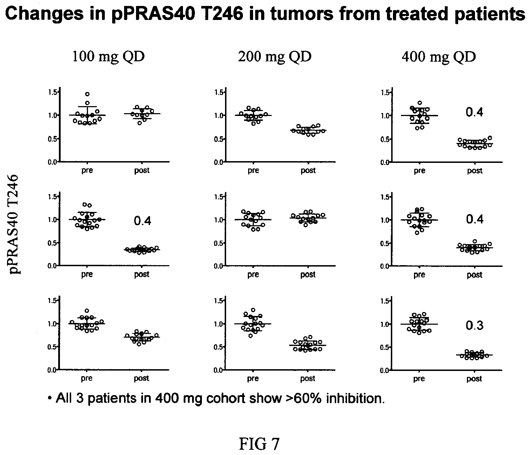

In certain embodiments of the invention, pGSK-3β or PRAS40 can be used as pharmacodynamic biomarkers for AKT inhibitors, such as GDC-0068. Further, in certain embodiments, the proper dosage of a compound, such as GDC-0068, can be determined, and adjusted based upon, inhibition of an AKT pathway, using PD biomarkers, *e.g.,* pGSK-3β or PRAS40 (*see* Figure 7).

It is also proposed that GDC-0068 and similar ATP competitive inhibitors are more active against hyperactivated Akt.

Preferential targeting of active Akt may act in concert with oncogene addition to increase the therapeutic index of GDC-0068 and similar ATP competitive inhibitors for tumors, with high steady state levels of active Akt, including those caused by Akt mutations, PTEN loss (either hemizygous or homozygous), INPP4B loss of function, PHLPP loss of function, PP2A loss of function, PI3K mutations and Her2 and/or Her3 amplification.

GDC-0068 efficacy is predicted in tumors that are PTEN null or have PI3k mutations, for example in prostate, breast and ovarian cancer.

PTEN loss is a biomarker that predicts synergy with MEK inhibitors, for example in pancreatic cancer.

Accordingly, GDC-0068 activity is associated, *e.g*., selectively associated, with AKT pathway activation. Evidence for this relationship was demonstrated in cell lines and xenograft studies.

PTEN loss, PI3K kinase domain mutations and high pAKT levels are important markers that predict a compound's activity, *e.g*., as a single agent; with additive effects with combinations of chemotherapeutic compouds, and with synergistic effects, *e.g*., with MEK inhibitors. Interestingly, synergy with a MEK inhibitor is seen with MEK pathway activation. Conversely, activation of MEK pathway (*e.g*., KRASBRAF) are markers of resistance to single agent activity (*e.g*., GDC-0068). Other potential predictors of a compound's activity, *e.g*., GDC-0068 activity, include RTK driven pathway activation (HER2 in breast, HER2 and Met in gastric cancer), AKT1 E17K mutations, AKT2 amplifications, AKT3 over-expression and PI3K amplifications.

**TABLE A Current estimates of prevalence of biomarkers for gastric, prostate and pancreatic cancer, based on reports from literature**

| | **Gastric** | **CRPC** | **Pancreatic** |
|---|---|---|---|
| PTEN Loss | 40% | 75-100% | 20-75% |
| PI3K Mutation | 8% | 2% | |
| PI3K Amplification | 36% | | |
| AKT Amplification | 20% | | 20% |
| KRAS Mutation | | | 90% |

For purposes of interpreting this specification, the following definitions will apply and whenever appropriate, terms used in the singular will also include the plural and vice versa. In the event that any definition set forth below conflicts with any document referred to herein, the definition set forth below shall control.

The term "polynucleotide" or "nucleic acid," as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (*e.g*., methyl phosphonates, phosphotriesters, phosphoamidates, cabamates, *etc.*) and with charged linkages (*e.g.,* phosphorothioates, phosphorodithioates, *etc.*)*,* those containing pendant moieties, such as, for example, proteins (*e.g*., nucleases, toxins, antibodies, signal peptides, poly-L-lysine, etc. ), those with intercalators (*e.g.,* acridine, psoralen, *etc.*)*,* those containing chelators (*e.g*., metals, radioactive metals, boron, oxidative metals, *etc.*)*,* those containing alkylators, those with modified linkages (*e.g.,* alpha anomeric nucleic acids, *etc.*)*,* as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α- anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR 2 ("amidate"), P(O)R, P(O)OR', CO or CH 2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, refers to short, single stranded polynucleotides that are at least about seven nucleotides in length and less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "primer" refers to a single stranded polynucleotide that is capable of hybridizing to a nucleic acid and allowing the polymerization of a complementary nucleic acid, generally by providing a free 3'-OH group.

The term "nucleotide variation" refers to a change in a nucleotide sequence (*e.g*., an insertion, deletion, inversion, or substitution of one or more nucleotides, such as a single nucleotide polymorphism (SNP)) relative to a reference sequence (*e.g*., a wild type sequence). The term also encompasses the corresponding change in the complement of the nucleotide sequence, unless otherwise indicated. A nucleotide variation may be a somatic mutation or a germline polymorphism.

The term "amino acid variation" refers to a change in an amino acid sequence (*e.g.*, an insertion, substitution, or deletion of one or more amino acids, such as an internal deletion or an N- or C-terminal truncation) relative to a reference sequence (*e.g*., a wild type sequence).

The term "detection" includes any means of detecting, including direct and indirect detection.

The term "diagnosis" is used herein to refer to the identification or classification of a molecular or pathological state, disease or condition. For example, "diagnosis" may refer to identification of a particular type of cancer, *e.g.,* a lung cancer. "Diagnosis" may also refer to the classification of a particular type of cancer, *e.g.,* by histology *(e.g.,* a non small cell lung carcinoma), by molecular features (*e.g*., a lung cancer characterized by nucleotide and/or amino acid variation(s) in a particular gene or protein), or both.

The term "prognosis" is used herein to refer to the prediction of the likelihood of cancer-attributable death or progression, including, for example, recurrence, metastatic spread, and drug resistance, of a neoplastic disease, such as cancer.

The term "prediction" or (and variations such as predicting) is used herein to refer to the likelihood that a patient will respond either favorably or unfavorably to a drug or set of drugs. In one embodiment, the prediction relates to the extent of those responses. In another embodiment, the prediction relates to whether and/or the probability that a patient will survive following treatment, for example treatment with a particular therapeutic agent and/or surgical removal of the primary tumor, and/or chemotherapy for a certain period of time without cancer recurrence. The predictive methods of the invention can be used clinically to make treatment decisions by choosing the most appropriate treatment modalities for any particular patient. The predictive methods of the present invention are valuable tools in predicting if a patient is likely to respond favorably to a treatment regimen, such as a given therapeutic regimen, including for example, administration of a given therapeutic agent or combination, surgical intervention, chemotherapy, *etc.*, or whether long-term survival of the patient, following a therapeutic regimen is likely.

The terms "cell proliferative disorder" and "proliferative disorder" refer to disorders that are associated with a measurable degree of abnormal cell proliferation. In one embodiment, the cell proliferative disorder is cancer.

"Tumor," as used herein, refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The terms "cancer" and "cancerous" refer to or describe the physiological condition in mammals that is typically characterized by unregulated cell growth and proliferation. Examples of cancer include, but are not limited to, carcinoma, lymphoma (*e.g*., Hodgkin's and non-Hodgkin's lymphoma), blastoma, sarcoma, and leukemia. More particular examples of cancers include squamous cell cancer, small-cell lung cancer, non-small cell lung cancer, adenocarcinoma of the lung, squamous carcinoma of the lung, cancer of the peritoneum, hepatocellular cancer, renal cell carcinoma, gastrointestinal cancer, gastric cancer, esophageal cancer, pancreatic cancer, glioma, cervical cancer, ovarian cancer, liver cancer, bladder cancer, hepatoma, breast cancer (*e.g*., endocrine resistant breast cancer), colon cancer, rectal cancer, lung cancer, endometrial or uterine carcinoma, salivary gland carcinoma, kidney cancer, liver cancer, prostate cancer, vulval cancer, thyroid cancer, hepatic carcinoma, melanoma, leukemia and other lymphoproliferative disorders, and various types of head and neck cancer.

The term "lung tumor" refers to any tumor of the lung, including but not limited to small-cell lung carcinoma and non-small cell lung carcinoma, the latter including but not limited to adenocarcinoma, squamous carcinoma, and large cell carcinoma.

The term "neoplasm" or "neoplastic cell" refers to an abnormal tissue or cell that proliferates more rapidly than corresponding normal tissues or cells and continues to grow after removal of the stimulus that initiated the growth.

A "lung tumor cell" refers to a lung tumor cell, either *in vivo* or *in vitro,* and encompasses cells derived from primary lung tumors or metastatic lung tumors, as well as cell lines derived from such cells.

As used herein, "treatment" (and variations such as "treat" or "treating") refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis.

An "individual," "subject" or "patient" is a vertebrate. In certain embodiments, the vertebrate is a mammal. Mammals include, but are not limited to, farm animals (such as cows), sport animals, pets (such as cats, dogs, and horses), primates (including human and non-human primates), and rodents (*e.g.,* mice and rats). In certain embodiments, a mammal is a human and can be either a male or female human.

An "effective amount" refers to an amount effective, at dosages and for periods of time necessary, to achieve the desired therapeutic or prophylactic result.

A "therapeutically effective amount" of a substance/molecule of the invention may vary according to factors such as the disease state, age, sex, and weight of the individual, and the ability of the substance/molecule, to elicit a desired response in the individual. A therapeutically effective amount encompasses an amount in which any toxic or detrimental effects of the substance/molecule are outweighed by the therapeutically beneficial effects. A "prophylactically effective amount" refers to an amount effective, at dosages and for periods of time necessary, to achieve the desired prophylactic result. Typically, but not necessarily, since a prophylactic dose is used in subjects prior to or at an earlier stage of disease, the prophylactically effective amount would be less than the therapeutically effective amount.

The term "long-term" survival is used herein to refer to survival for at least 1 year, 5 years, 8 years, or 10 years following therapeutic treatment.

The term "increased resistance" to a particular therapeutic agent or treatment option, when used in accordance with the invention, means decreased response to a standard dose of the drug or to a standard treatment protocol.

The term "decreased sensitivity" to a particular therapeutic agent or treatment option, when used in accordance with the invention, means decreased response to a standard dose of the agent or to a standard treatment protocol, where decreased response can be compensated for (at least partially) by increasing the dose of agent, or the intensity of treatment.

"Patient response" can be assessed using any endpoint indicating a benefit to the patient, including, without limitation, (1) inhibition, to some extent, of tumor growth, including slowing down or complete growth arrest; (2) reduction in the number of tumor cells; (3) reduction in tumor size; (4) inhibition (*e.g*., reduction, slowing down or complete stopping) of tumor cell infiltration into adjacent peripheral organs and/or tissues; (5) inhibition (*e.g*., reduction, slowing down or complete stopping) of metastasis; (6) enhancement of antitumor immune response, which may, but does not have to, result in the regression or rejection of the tumor; (7) relief, to some extent, of one or more symptoms associated with the tumor; (8) increase in the length of survival following treatment; and/or (9) decreased mortality at a given point of time following treatment.

"Antibodies" (Abs) and "immunoglobulins" (Igs) refer to glycoproteins having similar structural characteristics. While antibodies exhibit binding specificity to a specific antigen, immunoglobulins include both antibodies and other antibody-like molecules which generally lack antigen specificity. Polypeptides of the latter kind are, for example, produced at low levels by the lymph system and at increased levels by myelomas.

The terms "antibody" and "immunoglobulin" are used interchangeably in the broadest sense and include monoclonal antibodies (*e.g*., full length or intact monoclonal antibodies), polyclonal antibodies, monovalent antibodies, multivalent antibodies, multispecific antibodies (*e.g*., bispecific antibodies so long as they exhibit the desired biological activity) and may also include certain antibody fragments (as described in greater detail herein). An antibody can be chimeric, human, humanized and/or affinity matured.

"FOXO3a" refers to a forkhead/winged helix box class O protein that is a downstream target of the PI3K/AKT kinase signaling pathway. Activated AKT kinase directly controls the activity of FOXO3a through phosphorylation, leading to its translocation to the cytoplasm, where it is sequestered by the 14-3-3 chaperone protein. Inhibition of PI3K/AKT kinases leads to dephosphorylation and nuclear localization of FOXO3a, resulting in its activation. Nuclear localization of FOXO3a enables it to act as a transcription factor to induce cell cycle arrest and/or apoptosis through the up-regulation of its key target genes such as p27Kip1 and Bim.

"Localization profile" refers to the amount of a given molecule in a one location compared to the amount in a second location. In one example, a FOXO3a localization profile refers to the amount of FOXO3a in the cell nucleus compared to the amount in the cell cytoplasm. The localization profile can be expressed in terms of a ratio (*e.g*., amount of FOXO3a in nucleus divided by amount of FOXO3a in cytoplasm) or a subtraction (*e.g*., amount of FOXO3a in nucleus minus amount of FOXO3a in cytoplasm). A "nuclear localization profile" refers to a localization profile that is determined to have FOXO3a levels that are substantially higher in the nucleus than in the cytoplasm. In one example, a nuclear localization profile has greater than about 50% FOXO3a in the nucleus than in the cytoplasm. In other examples, a nuclear localization profile has greater than about 70%, alternatively greater than about 80%, alternatively greater than about 90% FOXO3a in the nucleus than in the cytoplasm. A "cytoplasmic localization profile" refers to a localization profile that is determined to have FOXO3a levels that are substantially higher in the cytoplam than in the nucleus. In one example, a cytoplasmic localization profile has greater than about 50% FOXO3a in the cytoplasm than in the nucleus. In other examples, a cytoplasmic localization profile has greater than about 70%, alternatively greater than about 80%, alternatively greater than about 90% FOXO3a in the cytoplasm than in the nucleus.

One aspect therefore includes a method of predicting the sensitivity of tumor cell growth to inhibition by a AKT kinase pathway inhibitor, comprising: determining the localization profile of FOXO3a in a tumor cell, wherein a cytoplasmic localization profile of FOXO3a correlates with sensitivity to inhibition by a AKT kinase inhibitor, and a nuclear localization profile of FOXO3a correlates with resistance to inhibition by a AKT kinase inhibitor.

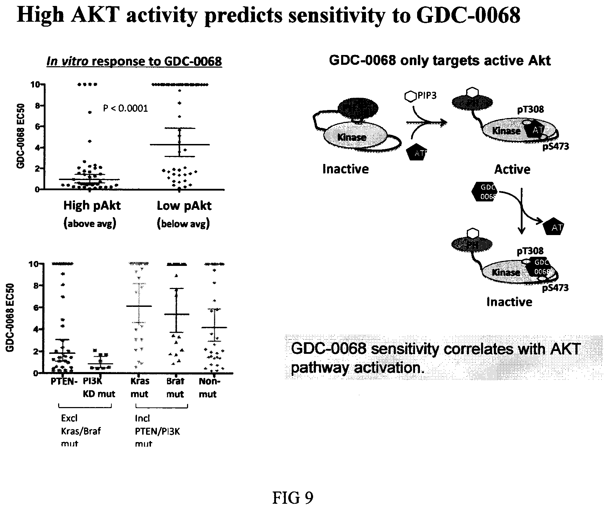

"pAKT profile" refers to the level of activation or phosphorylation of AKT ("pAKT") compared to the level of non-activated or non-phosphorylated AKT in a given sample. In one example, the sample is a tumor cell. The pAKT profile can be expressed in terms of a ratio (*e.g*., amount of pAKT in a tumor cell divided by amount of non-phosphorylated AKT in the cell or in a non-tumorous cell of the same type) or a subtraction (*e.g*., amount of pAKT in a tumor cell minus amount of non-phosphorylated AKT in the cell or in a non-tumorous cell of the same type). The pAKT profile can also be expressed in terms of the level of activation of the pathway by measuring amounts of phosphorylated downstream targets of AKT (for example, pGSK or PRAS40). A "high pAKT profile" refers to activation or phosphorylation levels of overall AKT in the sample that are higher than a baseline value. In one example, the baseline value is the basal levels of pAKT for a given cell type. In another example, the baseline value is average or mean level of pAKT in a given population of sample cells. In another example, a "high pAKT profile" refers to a tumor cell that overexpresses or has amplified phosphorylated or activated AKT in the cell, when compared to an average of normal, healthy (*e.g*., non-tumorous) cells of the same type from either the same mammal or a patient popluation. An example is shown in Figure 9 that demonstrates that a high pAKT profile predicts sensitivity to AKT inhibitors, for example GDC-0068. The pAKT profile can also be used in conjunction with other markers (for example PTEN loss, mutations to PI3K, Kras or Braf kinases, or FOXO3 localization profiles) for predicting efficacy of certain AKT inhibitors.

Methods of measuring levels of AKT activation and amounts of pAKT in a sample are known in the art. For example, immunoprecipitation assays can be used, such as the AKT Activity Assay Kit (available from Abcam^{®}, San Francisco, CA). In another example, Western blot assays can be used, such as the AKT Western Blot Assay Kit (available from Cell Signaling Technology, Danvers, MA). Other assay formats known for measuring pAKT levels include chemiluminescence-linked immunosorbent assays, *see* Cicenas, J, *et. al.,* "Increased level of phosphorylated akt measured by chemiluminescence-linked immunosorbent assay is a predictor of poor prognosis in primary breast cancer overexpressing ErbB-2," *Breast Can. Res.,* 7(4), R394, 2005. Other assays are available that can be used, for example the AlphaScreen SureFire Akt 1 (p-Thr308) Assay Kit (available from Perkin Elmer, Waltham, MA).

Methods of determining presence of PI3K mutations are known in the art. For example, assays for detection of specific mutations in the PIK3CA gene (in exons 9 and 20, and also H1047R or H1047L mutations), using real-time PCR are known (available from Qiagen, Valencia, CA).

A nucleic acid, may be *e.g*., genomic DNA, RNA transcribed from genomic DNA, or cDNA generated from RNA. A nucleic acid may be derived from a vertebrate, *e.g*., a mammal. A nucleic acid is said to be "derived from" a particular source if it is obtained directly from that source or if it is a copy of a nucleic acid found in that source.

Variations in nucleic acids and amino acid sequences may be detected by certain methods known to those skilled in the art. Such methods include, but are not limited to, DNA sequencing; primer extension assays, including allele-specific nucleotide incorporation assays and allele-specific primer extension assays (*e.g*., allele-specific PCR, allele-specific ligation chain reaction (LCR), and gap-LCR); allele-specific oligonucleotide hybridization assays (*e.g*., oligonucleotide ligation assays); cleavage protection assays in which protection from cleavage agents is used to detect mismatched bases in nucleic acid duplexes; analysis of MutS protein binding; electrophoretic analysis comparing the mobility of variant and wild type nucleic acid molecules; denaturing-gradient gel electrophoresis (DGGE, as in, *e.g.,* Myers *et al.* (1985) *Nature* 313:495); analysis of RNase cleavage at mismatched base pairs; analysis of chemical or enzymatic cleavage of heteroduplex DNA; mass spectrometry (*e.g*., MALDI-TOF); genetic bit analysis (GBA); 5' nuclease assays (*e.g*., TaqMan^{®}); and assays employing molecular beacons. Certain of these methods are discussed in further detail below.

Detection of variations in target nucleic acids may be accomplished by molecular cloning and sequencing of the target nucleic acids using techniques well known in the art. Alternatively, amplification techniques such as the polymerase chain reaction (PCR) can be used to amplify target nucleic acid sequences directly from a genomic DNA preparation from tumor tissue. The nucleic acid sequence of the amplified sequences can then be determined and variations identified therefrom. Amplification techniques are well known in the art, *e.g.,* polymerase chain reaction is described in Saiki et al., Science 239:487, 1988; U.S. Pat. Nos. 4,683,203 and 4,683,195.

The ligase chain reaction, which is known in the art, can also be used to amplify target nucleic acid sequences. *see, e.g.,* Wu et al., Genomics 4:560-569 (1989). In addition, a technique known as allele-specific PCR can also be used to detect variations (*e.g.,* substitutions). *see, e.g.,* Ruano and Kidd (1989) Nucleic Acids Research 17:8392; McClay et al. (2002) Analytical Biochem. 301:200-206. In certain embodiments of this technique, an allele-specific primer is used wherein the 3' terminal nucleotide of the primer is complementary to (*i.e.,* capable of specifically base-pairing with) a particular variation in the target nucleic acid. If the particular variation is not present, an amplification product is not observed. Amplification Refractory Mutation System (ARMS) can also be used to detect variations (*e.g.,* substitutions). ARMS is described, *e.g.,* in European Patent Application Publication No. 0332435, and in Newton et al., Nucleic Acids Research, 17:7, 1989.

Other methods useful for detecting variations (*e.g*., substitutions) include, but are not limited to, (1) allele-specific nucleotide incorporation assays, such as single base extension assays (*see, e.g.,* Chen et al. (2000) Genome Res. 10:549-557; Fan et al. (2000) Genome Res. 10:853-860; Pastinen et al. (1997) Genome Res. 7:606-614; and Ye et al. (2001) Hum. Mut. 17:305-316); (2) allele-specific primer extension assays (*see, e.g.,* Ye et al. (2001) Hum. Mut. 17:305-316; and Shen et al. Genetic Engineering News, vol. 23, Mar. 15, 2003), including allele-specific PCR; (3) 5'nuclease assays (*see, e.g.,* De La Vega et al. (2002) BioTechniques 32:S48-S54 (describing the TaqMan^{®} assay); Ranade et al. (2001) Genome Res. 11:1262-1268; and Shi (2001) Clin. Chem. 47:164-172); (4) assays employing molecular beacons (*see, e.g.,* Tyagi et al. (1998) Nature Biotech. 16:49-53; and Mhlanga et al. (2001) Methods 25:463-71); and (5) oligonucleotide ligation assays (*see, e.g.,* Grossman et al. (1994) Nuc. Acids Res. 22:4527-4534; patent application Publication No. US 2003/0119004 A1; PCT International Publication No. WO 01/92579 A2; and U.S. Pat. No. 6,027,889).

Variations may also be detected by mismatch detection methods. Mismatches are hybridized nucleic acid duplexes which are not 100% complementary. The lack of total complementarity may be due to deletions, insertions, inversions, or substitutions. One example of a mismatch detection method is the Mismatch Repair Detection (MRD) assay described, *e.g*., in Faham et al., Proc. Natl Acad. Sci. USA 102:14717-14722 (2005) and Faham et al., Hum. Mol. Genet. 10:1657-1664 (2001). Another example of a mismatch cleavage technique is the RNase protection method, which is described in detail in Winter et al., Proc. Natl. Acad. Sci. USA, 82:7575, 1985, and Myers et al., Science 230:1242, 1985. For example, a method of the invention may involve the use of a labeled riboprobe which is complementary to the human wild-type target nucleic acid. The riboprobe and target nucleic acid derived from the tissue sample are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full-length duplex RNA for the riboprobe and the mRNA or DNA. The riboprobe need not be the full length of the target nucleic acid, but can a portion of the target nucleic acid, provided it encompasses the position suspected of having a variation.

In a similar manner, DNA probes can be used to detect mismatches, for example through enzymatic or chemical cleavage. *see, e.g.,* Cotton et al., Proc. Natl. Acad. Sci. USA, 85:4397, 1988; and Shenk et al., Proc. Natl. Acad. Sci. USA, 72:989, 1975. Alternatively, mismatches can be detected by shifts in the electrophoretic mobility of mismatched duplexes relative to matched duplexes. *see, e.g.,* Cariello, Human Genetics, 42:726, 1988. With either riboprobes or DNA probes, the target nucleic acid suspected of comprising a variation may be amplified before hybridization. Changes in target nucleic acid can also be detected using Southern hybridization, especially if the changes are gross rearrangements, such as deletions and insertions.

Restriction fragment length polymorphism (RFLP) probes for the target nucleic acid or surrounding marker genes can be used to detect variations, *e.g*., insertions or deletions. Insertions and deletions can also be detected by cloning, sequencing and amplification of a target nucleic acid. Single stranded conformation polymorphism (SSCP) analysis can also be used to detect base change variants of an allele. *see, e.g.,* Orita et al., Proc. Natl. Acad. Sci. USA 86:2766-2770, 1989, and Genomics, 5:874-879, 1989.

The invention also provides a variety of compositions suitable for use in performing methods of the invention. For example, the invention provides arrays that can be used in such methods. In one embodiment, an array of the invention comprises individual or collections of nucleic acid molecules useful for detecting variations. For instance, an array of the invention may comprise a series of discretely placed individual allele-specific oligonucleotides or sets of allele-specific oligonucleotides. Several techniques are well-known in the art for attaching nucleic acids to a solid substrate such as a glass slide. One method is to incorporate modified bases or analogs that contain a reactive moiety that is capable of attachment to a solid substrate, such as an amine group, a derivative of an amine group, or another group with a positive charge, into nucleic acid molecules that are synthesized. The synthesized product is then contacted with a solid substrate, such as a glass slide coated with an aldehyde or other reactive group. The aldehyde or other reactive group will form a covalent link with the reactive moiety on the amplified product, which will become covalently attached to the glass slide. Other methods, such as those using amino propryl silican surface chemistry are also known in the art.

A biological sample, according to any of the above methods, may be obtained using certain methods known to those skilled in the art. Biological samples may be obtained from vertebrate animals, and in particular, mammals. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood. Variations in target nucleic acids (or encoded polypeptides) may be detected from a tumor sample or from other body samples such as urine, sputum or serum. Cancer cells are sloughed off from tumors and appear in such body samples. By screening such body samples, a simple early diagnosis can be achieved for diseases such as cancer. In addition, the progress of therapy can be monitored more easily by testing such body samples for variations in target nucleic acids (or encoded polypeptides). Additionally, methods for enriching a tissue preparation for tumor cells are known in the art. For example, the tissue may be isolated from paraffin or cryostat sections. Cancer cells may also be separated from normal cells by flow cytometry or laser capture microdissection.

### AKT KINASE INHIBITORS

Certain AKT kinase inhibitors are known as ATP-competitive inhibitors, for their ability to compete with ATP for binding to the active site of AKT. Certain AKT kinase inhibitors known as allosteric inhibitors do not bind to the active site of AKT. Also, AKT kinase inhibitors can be pan-AKT inhibitors, wherein the inhibitor can inhibit the activity of two or more of AKT-1, AKT-2 and AKT-3. AKT kinase inhibitors can be selective AKT inhibitors, wherein the inhibitor can inhibit the activity of one of AKT-1, AKT-2 and AKT-3, without inhibiting the activity of the other two.

In one embodiment, the AKT kinase inhibitor is (S)-2-(4-chlorophenyl)-1-(4-((5R,7R)-7-hydroxy-5-methyl-6,7-dihydro-5H-cyclopenta[d]pyrimidin-4-yl)piperazin-1-yl)-3-(isopropylamino)propan-1-one, or a pharmaceutically acceptable salt thereof.

### PREPARATION OF COMPOUND

The compound of the present invention may be prepared according to methods described in U.S. Patent Publication No. 2008/0051399 (U.S. Patent Appl. Ser. No. 11/773,949, filed July 5, 2007, entitled "Hydroxylated and Methoxylated Pyrimidyl Cyclopentanes as AKT Protein Kinase Inhibitors")

For illustrative purposes, Scheme 3 shows a general method for preparing the compound. Those skilled in the art will appreciate that other synthetic routes may be used. Although specific starting materials and reagents are depicted in the Scheme and discussed below, other starting materials and reagents can be easily substituted to provide a variety of derivatives and/or reaction conditions.

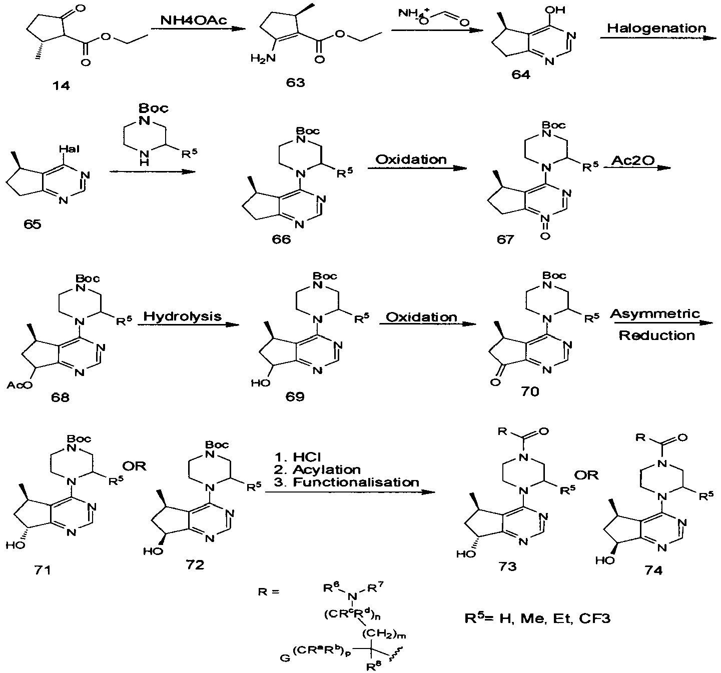

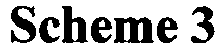

Scheme 3 shows a method of preparing compounds 73 and 74. According to Scheme 3, amination of 14 using an ammonia synthon gives 63. Pyrimidine formation using, for example, ammonium formate in the presence of formamide at 50°C-250°C and/or at high pressure gives the bicyclic unit 64. Activation of 64 using, for example, POCl₃ or SOCl₂ gives the activated pyrimidine 65. Displacement of this leaving group, using a suitable protected/substituted piperidine at 0°C to 150°C gives the piperidine 66. Oxidation, using, for example, m-chloroperoxybenzoic acid ("MCPBA" or "mCPBA") or Oxone^{®} at -20°C to 50°C gives the N-oxide 67. Treatment with an acylating agent (eg. acetic anhydride) followed by heating (40°C to 200°C) causes rearrangement to give 68. Hydrolysis, using, for example LiOH or NaOH at 0°C to 50°C gives the alcohol 69. Oxidation, using for example, Swern conditions, MnO₄ or pyridine-SO₃ complex at appropriate temperatures gives the ketone 70. Asymmetric reduction using, for example, a catalytic chiral catalyst in the presence of hydrogen, the CBS catalyst or a borohydride reducing agent in the presence of a chiral ligand gives rise to either the (R) or the (S) stereochemistry at the alcohol 71 or 72. Alternatively, a non-chiral reducing agent could be used (eg. H₂, Pd/C), allowing the methyl group on the cyclopentane unit to provide facial selectivity and ultimately diastereoselectivity. If the reduction gives a lower diastereoselctivity, the diastereomers could be separated by (for example) chromatography, crystallization or derivitization. Finally deprotection of the Boc-group, using, for example, acid at 0°C to 50°C, acylation using an appropriately functionalized amino acid and final functionalization of the amine of this amino acid (eg. removal of any protecting group, alkylation, reductive amination or acylation to introduce new substituents) gives rise to the final compounds 73 and 74.

Certain embodiments of the invention will now be illustrated by the following non-limiting Examples.

### Activating Mutations in AKT1

Applicants have discovered that certain mutations in *AKT1* can lead to disrupted interactions between the AKT1 PH domain and kinase domain. A disruption between these domains, caused by the mutation(s), appears to lead to constitutive phosphorylation of AKT1 and to constitutive AKT1 signaling. These effects also allow for the transformation of cells. These mutations confer resistance to PI3K and allosteric Akt inhibitors. Accordingly, the presence of such mutations indicate that the effective dosage for PI3K and allosteric Akt inhibitors will be higher, and also indicates that inhibitors other than PI3K and/or allosteric Akt inhibitors should be used, such as competitive Akt inhibitors.

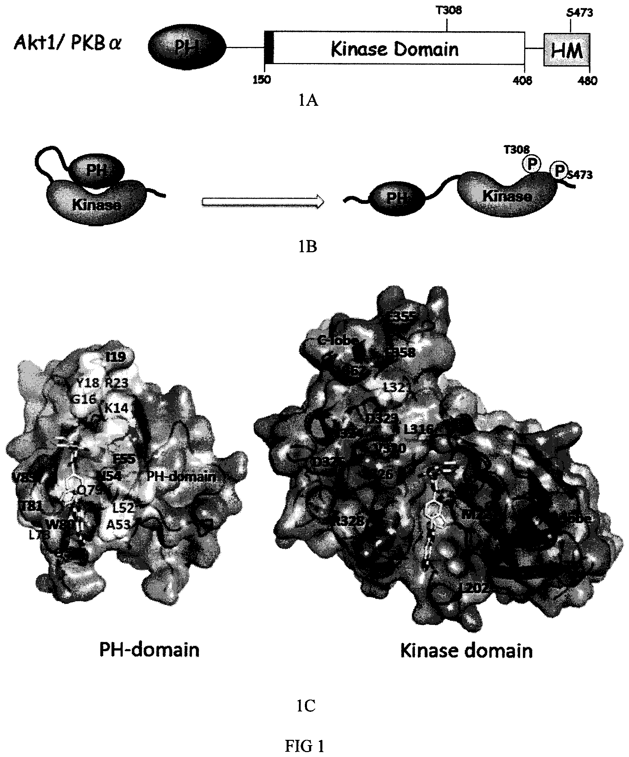

AKT, and the interactions of the PH and kinase domain, are depicted in Figure 1A and 1B. Applicants also present the locations of interactions between the PH and kinase domain in Figure 1C.

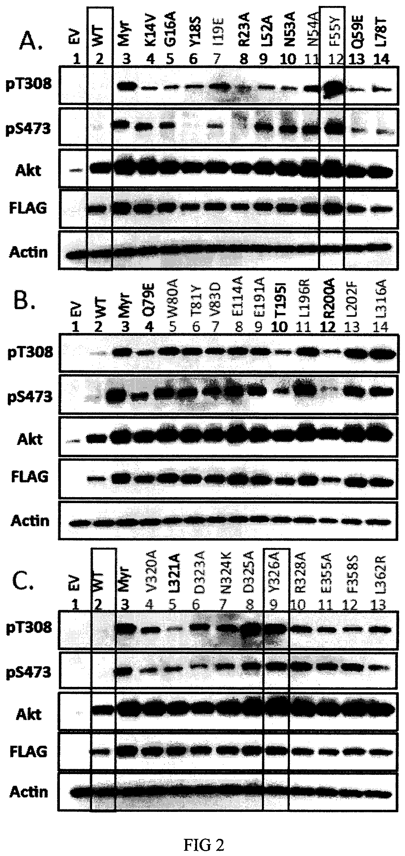

Results indicating that synthetic mutations at sites thought to disrupt the interactions of PH domain with the kinase domain lead to constitutive phosphorylation of Akt are shown in Figure 2.

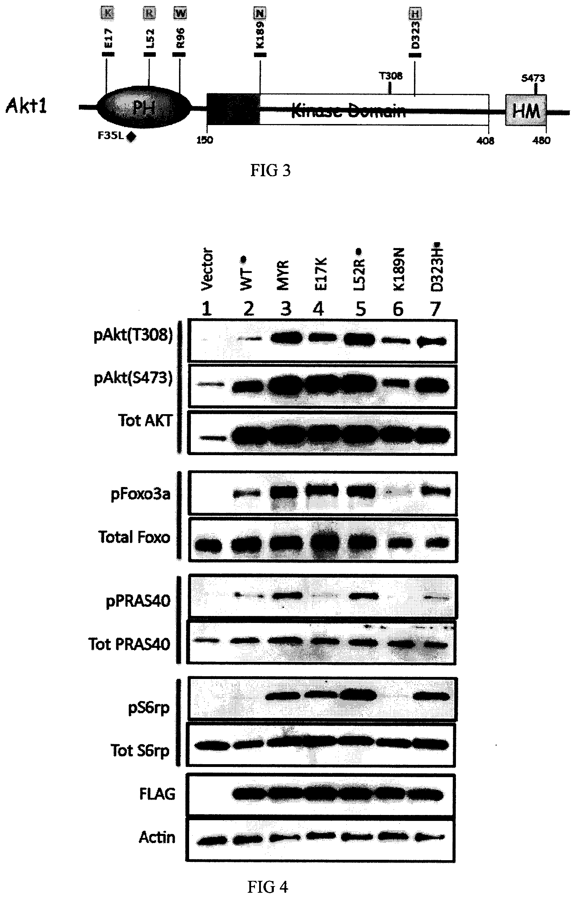

Somatic mutations found in cancer patients are shown in Figure 3.

Applicants have found that the somatic mutations lead to constitutive Akt phosphorylation and also lead to constitutive Akt signaling (*see* Figure 4).

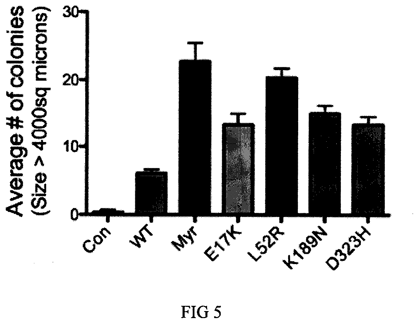

Applicants have also demonstrated that Akt1 mutants can transform cells (*see* Figure 5).

Interestingly, the Akt1 mutants confer resistance to PI3K inhibitors and to AKT allosteric inhibitors. This discovery of a biomarker that indicates what treatment regimens would be most beneficial to a patient that has such a biomarker, will be beneficial. Accordingly, these mutations are important for cancer diagnosis and for determining the proper therapeutic treatment.

Additionally, Akt mutations also affect Akt membrane localization, with minimal translocation to the plasma membrane.

### Mutations Leading to Constitutive Activation of AKT

The protein kinase AKT, a key regulator of cell survival and proliferation, is frequently hyperactivated in human cancers. Intramolecular pleckstrin homology (PH) domain-kinase domain (KD) interactions are important in maintaining AKT in an inactive state. AKT activation proceeds following a conformational change that dislodges the PH from the kinase domain. In order to understand these auto-inhibitory interactions, mutations at the PH-KD interface were generated, and it was found that a majority of them lead to constitutive activation of AKT. Such mutations are likely another mechanism by which activation may occur in human cancers and other diseases. In support of this, somatic mutations in *AKT1* at the PH-KD interface were found that have not been previously described in human cancers. Further, the *AKT1* somatic mutants are constitutively active, leading to oncogenic signaling. Additionally, the *AKT1* mutants are not effectively inhibited by allosteric AKT inhibitors, consistent with the need for an intact PH-KD interface for allosteric inhibition. These results have important implications for therapeutic intervention in patients with *AKT* mutations at the PH-KD interface.

In this study, a systematic analysis was performed to understand the effects of perturbing the PH-KD interactions on activation of AKT. Disrupting inter-domain contacts by mutating residues at the PH-KD interface lead to AKT activation. Given this, a large number of human tumors were sequenced to see if mutations at the PH-KD contact sites occur in cancers. Interestingly, it was found that human tumors that carry mutations in *AKT* at these sites, indicating disruption of the PH-KD interactions, is an important mechanism for AKT activation in cancers. These tumor specific somatic mutations were tested for activity and it has been shown that they are oncogenic. Also, AKT inhibitors were tested against these mutants, and the ATP-competitive inhibitors are more effective compared to allosteric AKT inhibitors.

### Perturbing PH-kinase domain contacts lead to AKT activation

To assess the activation status of AKT, an assay was developed that measured the ability of AKT to promote growth factor independent survival of IL-3-dependent BaF3 cells. The BaF3 pro-B-cells can be rendered growth factor independent by enforced expression of oncogenes. BaF3 cells expressing wildtype AKT1 (WT AKT1), Myristoylated (Myr) or the E17K AKT1 mutant were generated and it was found that activated AKT by itself was unable to promote factor independence. However, co-expression of Myr AKT1 or the oncogenic E17K AKT1 and an activated form of the MAP2 kinase MEK1 (Mek1 ΔN3, S218E, S222D) promoted factor independent growth and survival of BaF3 cells. Although WT AKT1 in combination with active MEK1 (MEK1 N3) showed some activity in this assay, it was less effective compared to mutant AKT1.

The BaF3 assay was used to investigate the consequence of disrupting PH-KD interactions. Using the recently published full-length structure of AKT1 (Wu et al., (2010). PLoS One 5, e12913), residues were identified at the PH-KD interface. Mutations at these sites were designed to compromise the PH-KD interaction by removal of favorable interactions, increasing steric bulk or reversing the charge of side chains involved in inter-domain polar contacts. A library of 35 such *AKT1* mutants was generated (Figure 16; Table 1). Also included in the pool were an AKT1 E17K mutant construct that served as a positive control and a WT AKT1 clone with a silent mutation that served as a negative control for activity. Three AKT mutant library was used to derive a pool of BaF3 cells that stably co-expressed the mutants along with MEK1 N3. After allowing growth in the absence of IL-3, the pool of cells was sampled at 3 days and 4 days post IL-3 withdrawal and the proportion of various mutants in the pool determined relative to the input at 0 hours, using next-generation sequencing (Figure 16C). Each mutation was scored based on a normalized ratio of observed frequency at a given time point compared to the input frequency and these ratios were then normalized to the ratios for WT AKT1. As expected, AKT1 E17K was more than 50 times enriched over wild type. Similarly, mutants such as T81Y and D323A were also strongly enriched (>15 fold over WT) indicating that these mutations lead to AKT activation. Other mutants, R23A, N53A, F55Y, L78T, Q79E, W80A, E191A, T195I, V270A, V271A, L321A, D325A and R328A showed moderate enrichment (2-6 fold over WT at either the 3 day or the 4 day time-point) in the assay, and are likely activating (Figure 16D, Table 1).

**Table 1 - Summary of AKT1 PH-KD mutations and their effects**

| **AKT1 mutation** | **Residue Location** | **Activation Status (pT308/pS473 in NIH3T3)** | **BaF3 survival assay** | **Key inter-domain Interactions in WT** |
|---|---|---|---|---|
| WT (L52L) | negative control | - | - | n/a |
| Myr (K189K) | positive control | + | + | n/a |
| E17K | positive control | nd | + | close to D323 pocket |
| m | inter-domain contact* | + | - | polar contact with D323 |
| G16A | interface edge | + | - | close to D323 pocket |
| Y18S | inter-domain contact* | + | - | polar and hydrophobic contacts to R273, K297, V320 & Y326 |
| I19E | inter-domain contact* | + | - | hydrophobic contacts with L321 & F358 |
| R23A | inter-domain contact* | - | + | polar contact with D323 |
| L52A | inter-domain contact* | + | - | hydrophobic contacts with V270, V271 and Y326 |
| N53A | interface edge | + | + | close to N269 & V270 |
| N54A | inter-domain contact* | + | - | hydrogen bonds to interfacial water; close to V271 & Y326 |
| F55Y | inter-domain contact* | + | + | hydrophobic contacts with Y326 |
| Q59E | interface edge | - | - | likely close to 188-198 loop |
| L78T | inter-domain contact* | + | + | hydrophobic contacts with L181, L223 & F225 |
| Q79E | interface edge | + | + | close to allosteric inhibitor pocket |
| W80A | inter-domain contact* | + | + | hydrophobic contacts with V201 & L213 |
| T81Y | inter-domain contact* | + | + | hydrophobic contact to F225; HB to D292 |
| V83D | inter-domain contact* | + | + | hydrophobic contact with F161, L181 & I186 |
| E114A | interface edge | + | - | part PH-KD linker (loop not defined in structure) |
| E191A | interface edge | + | - | part of 188-198 loop in KD missing in structure |
| T195I | interface edge | + | + | part of 188-198 loop in KD missing in structure |
| L196R | interface center | + | - | part of 188-198 loop in KD missing in structure |
| R200A | interface edge | + | - | close to W80 |
| L202F | inter-domain contact* | + | - | hydrophobic contact with W80 |
| V270A | inter-domain contact* | nd | + | hydrophobic contacts with L52 & N53 |
| V271A | inter-domain contact* | nd | + | hydrophobic contacts with L52 |
| L316A | inter-domain contact* | + | - | hydrophobic contact with Y18 |
| V320A | inter-domain contact* | + | + | hydrophobic contact with Y18 |
| L321A | inter-domain contact* | + | + | hydrophobic contact with Y18 |
| D323A | inter-domain contact* | + | + | polar contact with K14, R23 & R25 |
| N324K | inter-domain contact* | + | + | hydrogen bond contact with R25 |
| D325A | interface edge | + | + | hydrogen bond to K39 bb and interfacial water |
| Y326A | inter-domain contact* | + | + | hydrophobic contact with F55 |
| R328A | interface edge | + | + | close to L52 |
| E355A | interface edge | + | - | close to I19 |
| F358S | inter-domain contact* | + | - | hydrophobic contact with I19 |
| L362R | interface edge | + | - | close to I19 |

| | | | | |
|---|---|---|---|---|
| * Residues identified as inter-domain contacts from crystal structure of AKT1 (Wu et al, Plos One, 2010; PDB Accession Code 3O96) nd = not determined | | | | |

To further understand the effect of PH-KD interface mutants, NIH3T3 cell lines were generated stably expressing each of the *AKT1* mutants and assessed the T308 and S473 phosphorylation status (pT308 and pS473). Consistent with the survival assay screen, N53A, F55Y, L78T, Q79E, W80A, T81Y, E191A, T195I, L321A, D323A, D325A and R328A mutants showed elevated phosphorylation on T308 and S473 (Figure 16E). Further, although *AKT1* mutants N54A, V83D, E114A, L202F, V320A, N324K and Y326A showed only a mild enrichment (~1.5-2 fold over WT) in the survival screen, they showed elevated levels of pT308 and pS473. These results indicate that disrupting the PH-KD contacts lead to constitutive phosphorylation of AKT.

### Identification of AKT somatic mutations in cancer

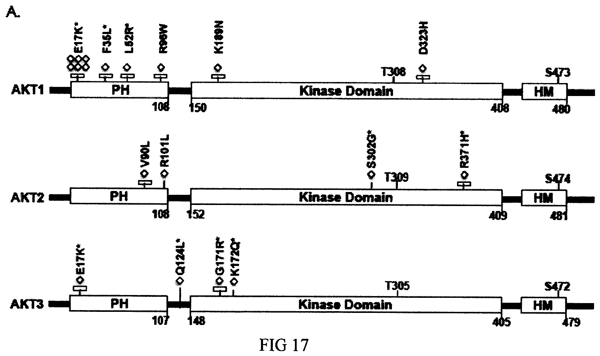

Given that perturbation of the PH-KD interface led to AKT activation, it was assessed if such mutations occur in human primary tumors. To identify potential *AKT* mutations, all the coding exons of *AKT1,* 2 and 3 were sequenced in a total of 394 human primary tumor samples consisting of 65 colorectal, 51 breast, 48 non-small-cell lung (NSCLC) adenocarcinoma (adeno), 43 NSCLC (squamous), 43 renal carcinoma, 37 melanoma, 33 gastric, 32 ovarian, 15 esophageal, 11 hepatocellular (HCC), 10 small-cell lung cancer (SCLC) and 6 others (5 lung large cell and 1 lung cancer other). Protein-altering, somatic *AKT1* mutations were found in 4 % of breast (2/51) and 1.5 % of colon (1/65). *AKT2* somatic mutations were found in ~5% of NSCLC (adeno; 2/43) (Figure 17).

Besides the E17K mutation, there is also an *AKT1* mutation at codon 52, L52R. L52 is at the PH-KD interface and makes hydrophobic contacts with V270, V271, Y326 and the methylene portion of R328 in the kinase domain. Although the L52A mutation increased the level of AKT1 phosphorylation compared to WT, it did not lead to an increase in survival in the BaF3 assay. However, L52R mutation is likely to be deleterious to PH-KD interactions (and hence promote AKT1 activity) since the favorable hydrophobic interactions will be lost and an unfavorable interaction with R328 introduced. Similarly, the D323H mutation identified in breast tumor is located in the AKT1 kinase domain and is proximal to three basic residues in the PH domain (K14, R23 and R25) (Figure 1B, 2B). The synthetic mutant D323A was constitutively active, suggesting that D323H will also be constitutively active since a histidine is even more disruptive to the inter-domain contacts than alanine (increased steric bulk; potential for charge reversal). The R96 residue is located in the main PH domain helix and is far removed from the kinase domain interface and hence it is unlikely to promote activation through disruption of PH-KD interactions. Electron density is not observed for residues K189 to E198 in the full-length AKT1 crystal structure. Although this kinase domain loop is likely proximal to the C-terminal end of the PH domain helix, a structure-based estimate of AKT1 activity changes associated with the K189N mutation is not possible.

**Table 2: AKT family protein altering mutations in human cancers**

| **Gene** | **Tumor** | **Mutations (amino acid change)** |
|---|---|---|
| AKT1 | Breast Ca | E17K |
| AKT1 | NSCLC (Adeno) | F35L, E17K |
| AKT1 | Breast Cancer (HR+) | L52R, E17K |
| AKT1 | Melanoma | E17K |
| AKT1 | Endometrial Ca | E17K |
| AKT1 | Prostate | E17K |
| AKT1 | Bladder Ca (tumor and cell lines) | E17K |
| AKT1 | Bladder Ca (cell line) | E49K |
| AKT2 | Colon Ca | S302G, R371H |
| AKT3 | NSCLC (Adeno) | Q124L, |
| AKT3 | Glioma | G171R |
| AKT3 | Leukemia (CLL) | K172Q |
| AKT3 | Melanoma (tumor and cell lines) | E17K |

### AKT1 somatic mutants signal constitutively and lead to transformation

To assess the functional relevance of the AKT1 somatic mutations, the L52R and D323H mutants that are predicted to affect PH-KD interactions were tested. In addition, the K189N mutation that occurs in the kinase domain was tested. These mutants were tested for their effect on signaling, using NIH3T3 cells stably expressing N-terminally FLAG-tagged AKT1 WT, Myristoylated AKT1 (Myr AKT1), or the mutants E17K, L52R, K189N and D323H. Immunoblot analysis showed that unlike the vector transduced or the AKT1 WT cells, all the mutants except for K189N, showed an increase in both pT308 and pS473, similar to Myr AKT1. Consistent with this we observed a concomitant increase in phosphorylation of the AKT substrates FOXO and S6 ribosomal protein in cells expressing Myr or mutant AKT1 compared to cells expressing empty vector, WT AKT1, or the K189N AKT1 mutant. Interestingly, unlike Myr AKT or the L52R mutant, expression of E17K or D323H did not result in elevated phosphorylation of the AKT substrate PRAS40, suggesting that these mutants may engage different downstream effector pathways.

### AKT1 PH-kinase domain mutants weaken inter-domain interactions and show impaired plasma membrane translocation

Although the cancer specific *AKT1* mutations, L52R and D323H, occur at positions predicted to disrupt the PH-KD interactions, the amino acid substitutions observed were different from the synthetic mutants generated and analyzed earlier. To directly test whether these somatic mutations weaken inter-domain interactions, a mammalian 2-hybrid assay was performed using AKT PH and KD constructs fused to the VP16 activation domain (VP16 AD) and Gal4 DNA binding domain (Gal4 DBD), respectively. The strength of the interaction was measured using a luciferase reporter where the luciferase activity is proportional to the strength of the interaction. The L52R PH domain/WT-KD and the WT-PH/D323H KD combination showed a 50% reduction in the interaction signal compared to WT-PH/WT-KD or the E17K-PH/WT-KD, confirming that the L52R and D323H mutants are deficient in the PH-KD interaction.

To further understand the mechanism of activation of *AKT* mutants, their cellular localization was determined. In resting cells, wild type AKT1 is diffusely localized throughout the cytoplasm and nucleus and in response to mitogenic stimulation is rapidly translocated to the plasma membrane, leading to its activation.

The L52R PH domain mutant was tested for sub-cellular localization and membrane translocation using a GFP-AKT1 PH domain fusion construct. WT and E17K AKT1 PH domain GFP fusion constructs served as controls. In the absence of growth factor stimulation, while the WT AKT1 PH domain was distributed throughout the cytoplasm and nucleus, the E17K AKT1 PH domain was constitutively localized to the plasma membrane. In contrast to the E17K AKT1 PH domain, the L52R AKT1 PH domain was distributed throughout the cytoplasm and nucleus, behaving like the WT AKT1 PH domain. However, upon growth factor stimulation, unlike the WT AKT1 PH domain, the mutant L52R PH domain did not translocate to the plasma membrane. This suggests that unlike the E17K mutant which is activated in response to altered lipid affinity and localization, the L52R mutant is most likely activated in the cytoplasm, due to absence of auto-inhibitory interactions.

### Disruption of AKT2 and AKT3 PH-KD interactions lead to their activation

Given the common domain architecture of the AKT family members, whether disrupting the PH-KD interactions in AKT2 and AKT3 can lead to their activation was tested. To test this, *AKT2* mutants L52R and D324H and *AKT3* mutants L51R and D320H (equivalent of AKT1 L52R and D323H) were generated, all of which are predicted to disrupt the PH-KD interaction. Since *AKT3* E17K mutations can be found in melanoma, and *AKT2* E17K mutation in human hypoglycemina have been reported, the E17K *AKT2* and *AKT3* mutants were generated, along with additional *AKT2* and *AKT3* somatic mutations that have been identified in human cancers. In addition, Myr AKT2 and Myr AKT3 were generated as positive controls. The *AKT2* and *AKT3* mutants were stably expressed in NIH3T3 cells and the phosphorylation status of T308 and S473 was assessed. AKT2 E17K, L52R and D324H, and AKT3 E17K, L51R and D320H all showed elevated pT308 and pS473 compared to the WT AKT2 or AKT3 expressing cells. Consistent with the activation status, these AKT2 and AKT3 mutants were able to support growth factor independent survival of BaF3 cells in combination with activated MEK1. Interestingly, the AKT2 mutant R371H identified in human cancer also showed elevated pT308 and pS473, but was not capable of promoting growth factor independent survival of BaF3. The remaining mutants (AKT2 V90L and R101L and AKT3 Q124L and G171R) did not show an increase in pT308 and pS473 and were not able to support growth factor independent survival of BaF3 cells. Inspection of the homology models generated for full length AKT2 and AKT3 indicate that these mutations all occur in surface-exposed loops and are not proximal to the PH-KD interface. Note that even though AKT2 V90L and AKT3 Q124L are in loops not defined in the AKT1 electron density, the termini of these loops are not proximal to the PH-KD interface. Thus, the structural analysis does not offer any insight into how AKT2 R371H is able to elevate phosphorylation, or how any of these mutants might be a driving force for the cancers in which they were identified.

### AKT1 somatic mutants promote oncogenesis in vivo

Previous studies have shown that BaF3 cells stably expressing oncogenes, when implanted in mice, promote a leukemia-like disease, leading to reduction in overall survival. Since the AKT1 mutants co-operate with active MEK1 to promote factor-independent growth of BaF3 cells, this model system was used to test their tumorigenic potential *in vivo.* Mice implanted with BaF3 cells co-expressing MEK1 N3 and Myr AKT1 or mutant AKT1 E17K, L52R or D323H, showed a median survival of 19 to 20.5 days. In contrast, mice that received BaF3 cells co-expressing MEK1 N3 and AKT1 WT have a significantly longer median survival of 29 days. This is consistent with the fact that AKT1 WT in the context of active MEK1 was able to support factor-independent survival of BaF3 cells, though the effect was modest compared to the AKT mutants. As expected, mice receiving control BaF3 cells that expressed MEK1 N3 alone were alive at the end of the 55-day study period. Necropsies were performed at 19 days post-transplantation, on a cohort of 3 mice per treatment group to follow disease progression. Consistent with the reduced overall survival, a significant proportion of GFP tagged BaF3 cells were found in the bone marrow and spleens of mice that received mutant AKT1, compared to mice that received WT AKT1 or vector control cells. A significant enlargement of liver and spleen in mice expressing mutant AKT1 was found. Histological examination of hematoxylin and eosin (H&E) stained liver, spleen, and bone marrow sections showed evidence of infiltration with leukemic blasts in mice that received mutant AKT1 transduced cells as compared to those that received vector control or WT AKT1 cells. These results confirm the transforming potential of the AKT1 mutants *in vivo.*

### AKT1 PH-KD interaction deficient mutants are less sensitive to allosteric inhibitors

Several ATP-competitive and allosteric small molecule inhibitors of AKT are in development and/or clinical trials (Mattmann et al., (2011). Expert Opin Ther Pat.; Pal et al., (2010). Expert Opin Investig Drugs 19, 1355-1366) Previous studies have shown that allosteric inhibitors of AKT require the presence of an intact PH-KD interface for their activity. Given that some AKT1 somatic mutants have impaired PH-KD contacts, it was predicted that allosteric inhibitors are likely to be less efficacious in inhibiting the activity of these mutants. The activity of two ATP-competitive inhibitors (GNE-692 Bencsik et al. (2010). Bioorg Med Chem Lett 20, 7037-7041) and GSK690693 (Rhodes et al. (2008). Cancer Res 68, 2366-2374) and two allosteric inhibitors (Inhibitor VIII (Lindsley et al., (2005). Bioorg Med Chem Lett 15, 761-764) and GNE-929 were tested on recombinant full length WT and mutant AKT1 enzymes. The effect of the inhibitors on the proliferation of NIH3T3 cells expressing WT or AKT1 mutants was also tested.

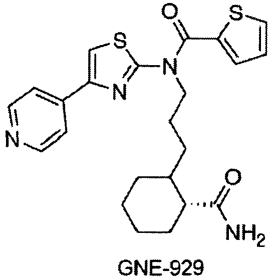

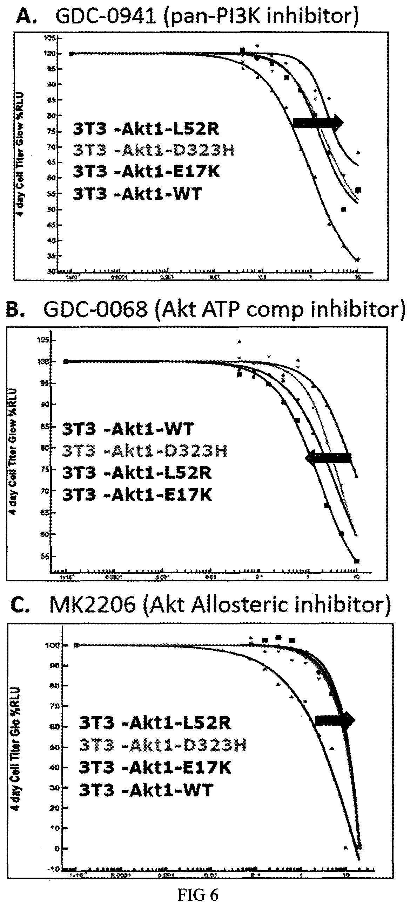

In biochemical activity assays, the ATP-competitive inhibitors GNE-692 and GSK690693 were effective in blocking the activity of the WT AKT1 enzyme (GNE-692 IC₅₀ 24.3nM) as well as the mutant enzymes (E17K, L52R and D323H; GNE-692 IC₅₀ 3.7-15.8 nM). Similarly, the ATP-competitive inhibitors were equally effective against both WT and mutant AKT1 in the cell based proliferation assay. In contrast, the allosteric inhibitors, Inhibitor VIII and GNE-929 were less effective against recombinant full length mutant enzymes (Inhibitor VIII: IC₅₀ 268.4nM for L52R; IC₅₀>1µM for D323H) compared to WT AKT1 (Inhibitor VIII: IC₅₀ 119.3nM; Figure 6C). Consistent with this, in a cell-based assay the allosteric inhibitor, Inhibitor VIII was found to be at least 50% less effective at blocking proliferation of cells expressing mutant AKT1 compared to WT AKT.

To confirm that the reduced sensitivity of the mutants to allosteric inhibitors was due to the impaired PH-KD interactions, an *in vitro* biochemical reconstitution assay was performed using purified recombinant PH and kinase domains. In this system, allosteric Inhibitor VIII when assayed against AKT1 kinase domain alone was unable to block its activity. However, when purified WT PH domain was added to the kinase domain and reconstituted the enzyme, Inhibitor VIII was able to block the activity of the enzyme, though there was a three fold increase in IC₅₀ for the reconstituted enzyme (IC₅₀ 238.8nM) compared to the full length WT enzyme (IC₅₀ 80.8nM). In contrast, reconstitution with mutant PH domain (L52R or E17K) further impaired the ability of Inhibitor VIII in blocking AKT1 (L52R IC₅₀ 713.5nM and E17K IC₅₀ >1µM). Similarly, Inhibitor VIII showed no activity when the WT PH domain was reconstituted with a mutant D323H kinase domain. The lack of E17K inhibition by allosteric inhibitors suggests that in addition to increased affinity for PIP2 this mutation may also affect the PH-KD interaction leading to its activation. In the cell based 2-hybrid assay the E17K mutant did not reveal a defect in PH-KD interaction indicating that the exact mechanism of E17K activation requires further investigaton. These data confirm the importance of an intact PH-KD interface for AKT1 allosteric inhibitors.

### Surrogate PD biomarker assays

Phospho-GSK-3b in Platelet rich plasma (PRP) was used as a surrogate PD biomarker to measure Akt pathway inhibition in patients after treatment with GDC-0068 at different time points over 22 days. Peripheral blood was collected in Vacutainer containing .38% of citrate as anti-coagulant. Blood was spun at 200 g for 15 min at room temperature. The PRP layer was carefully taken from the tube and then lysed in a buffer containing detergents, protease and phosphatase inhibitors. Phosphorylated and total GSK-3β levels in PRP lysates were measured using a phospho-GSK3 β /total-GSK3β multiplexed MSD assay. pGSk-3β levels were normalized to total GSk-3β levels and post-dose inhibition of pGSk-30 was expressed as a ratio of the pre-dose levels for each patient. A dose- and time-dependent pharmacologic response was demonstrated, with a decrease in pGSK3β level of ≥ 75% at doses ≥ 200 mg.

### Reverse Phase Protein Arrays

Core-needle tumor biopsies from patients treated with GDCC0068 were flash-frozen in OCT and sectioned into 8 um slices. Tissue was lysed in RPPA lysis buffer containing TPER, 300 mM NaCl and phosphatase inhibitors. Phosphoprotein signatures of the lysates were analysed using Reverse-Phase protein arrays: samples were printed on nitrocellulose slides and stained with Sypro to determine total protein concentrations. Each slide was stained with a different antibody at 4°C overnight. The data was then normalized to total protein levels and spatial effects were removed using Quadrant median normalization. Decreases of 60%-70% in pPRAS40 and ~50% decrease in Cyclin D1 (compared with baseline) occurred in all 3 patients treated at 400 mg daily. For methods and overview of RPPA *see*: Reverse phase protein microarrays advance to use in clinical trials, Molecular Oncology. 2010 Dec;4(6):461-81, Mueller C *et al.* In certain embodiments, the cancer to be treated herein comprises one or more of AKT, PI3k, PTEN and HER2 mutations or AKT, PI3k, PTEN or HER2 abberant signaling. In one example, the cancer is gastric cancer comprising high pAKT activity and PTEN low or null status. While certain embodiments of invention are described, and many details have been set forth for purposes of illustration, certain of the details can be varied without departing from the basic principles of the invention.

The use of the terms "a" and "an" and "the" and similar terms in the context of describing embodiments of invention are to be construed to cover both the singular and the plural, unless otherwise indicated herein or clearly contradicted by context. The terms "comprising," "having," "including," and "containing" are to be construed as open-ended terms (*i.e.,* meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. In addition to the order detailed herein, the methods described herein can be performed in any suitable order unless otherwise indicated herein or otherwise clearly contradicted by context. The use of any and all examples, or exemplary language (*e.g*., "such as") provided herein, is intended merely to better illuminate embodiments of invention and does not necessarily impose a limitation on the scope of the invention unless otherwise specifically recited in the claims. No language in the specification should be construed as indicating that any non-claimed element is essential to the practice of the invention.

## Claims

1. A method of predicting the sensitivity of the growth of a tumor cell to an AKT inhibitor, comprising determining the presence of a mutation to AKT, wherein the presence of a mutation to AKT correlates with sensitivity of the cell to the AKT inhibitor, wherein the AKT inhibitor is (S)-2-(4-chlorophenyl)-1-(4-((SR,7R)-7-hydroxy-5-methyl-6,7-dihydro-5H-cyclopenta[d]pyrimidin-4-yl)piperazin-1-yl)-3-(isopropylamino)propan-1-one, or a pharmaceutically acceptable salt thereof, wherein the mutation corresponds to, or aligns with, L52, E17, or Q79.

2. The method of claim 1, wherein the cell shows increased sensitivity to the AKT inhibitor.

3. The method of claim 1 or 2, wherein the AKT is AKT1, AKT2 or AKT3

4. The method of claim 3, wherein the AKT is AKT1.

5. The method of any one of claims 1 to 4, wherein the mutation corresponds to, or aligns with, E17 or Q79.

6. The method of any one of claims 1 to 4, wherein:
a. the L52 mutation corresponds to, or aligns with, L52R; and/or
b. the E17 mutation corresponds to, or aligns with, E17K; and/or
c. the Q79 mutation corresponds to, or aligns with, Q79E.

7. The method of claim 4, comprising determining the presence of a mutation to AKT1, wherein:
a. the mutation corresponds to, or aligns with, L52R; or
b. the mutation corresponds to, or aligns with, E17K; or
c. the mutation corresponds to, or aligns with, Q79E.

8. The method of any one of claims 1 to 7, wherein the mutation to AKT: (i) disrupts the interaction between the PH-domain and the kinase domain of AKT; or (ii) increases phosphorylation of AKT, and optionally wherein the increase in phosphorylation is due to constitutive phosphorylation.

9. The method of any one of claims 1 to 8, wherein the method further comprises determining the PTEN status of the cell, wherein PTEN loss correlates with increased sensitivity to the inhibitor.

10. The method of any one of claims 1 to 9, wherein the tumor cell is a prostate cancer cell, an ovarian cancer cell, a breast cancer cell, a gastric cancer cell, or a pancreatic cancer cell; or wherein the tumor cell is an endocrine resistant breast cancer cell.

11. The method of any one of claims 1 to 10, wherein the method further comprises determining the presence of a PI3K mutation of the cell, wherein the presence of a PI3K mutation correlates with increased sensitivity to inhibition by the inhibitor, optionally wherein said PI3K mutations are one or both of H1047R and H1047L.

12. The method of any one of claims 1 to 11, wherein the method further comprises determining the pAKT profile of the cell, wherein: (i) a high activity profile for pAKT correlates with increased sensitivity to inhibition by the inhibitor; or (ii) a low activity profile for pAKT correlates with decreased sensitivity to inhibition by the inhibitor.

13. The method of any one of claims 1 to 12, wherein the method further comprises: (i) determining the Her2 and/or Her3 status of the cell, wherein a Her2+ and/or Her3+ status of the cell correlates with increased sensitivity to inhibition by the inhibitor; or
(ii) determining the presence of a Met kinase mutation of the cell, wherein the presence of a Met kinase mutation correlates with sensitivity of the cell to the AKT inhibitor; or
(iii) determining the presence of a B-Raf or K-Ras mutation of the cell, wherein the presence of a B-Raf or K-Ras mutation correlates with a decrease in sensitivity of the cell to the AKT inhibitor; or
(iv) determining the localization profile of FOXO3a in the cell, wherein a cytoplasmic localization profile of FOXO3a correlates with sensitivity of the cell to inhibition by the Akt inhibitor; or
(v) determining the INPP4B, PHLPP, and/or PP2A status of the cell,
wherein INPP4B, PHLPP, and/or PP2A loss correlates with increased sensitivity to the inhibitor.

14. The method of any one of claims 1 to 13, further comprising determining the presence of the mutation to AKT in a physiological sample from a patient, and optionally further comprising informing a patient for whom the presence of a mutation to AKT is determined that an AKT inhibitor should be administered or should not be administered.

15. (S)-2-(4-chlorophenyl)-1-(4-((5R,7R)-7-hydroxy-5-methyl-6,7-dihydro-5H-cyclopenta[d]pyrimidin-4-yl)piperazin-1-yl)-3-(isopropylamino)propan-1-one, or a pharmaceutically acceptable salt thereof, for use in a method of treating a cancer cell comprising one or more AKT mutation, wherein the mutation corresponds to, or aligns with, L52, E17, or Q79.

## Patentansprüche

1. Verfahren zur Vorhersage der Empfindlichkeit des Wachstums einer Tumorzelle auf einen AKT-Inhibitor, umfassend das Bestimmen der Gegenwart einer Mutation von AKT, wobei die Gegenwart einer Mutation von AKT mit der Empfindlichkeit der Zelle auf den AKT-Inhibitor korreliert, wobei der AKT-Inhibitor (S)-2-(4-Chlorphenyl)-1-(4-((5R,7R)-7-hydroxy-5-methyl-6,7-dihydro-5H-cyclopenta[d]pyrimidin-4-yl)piperazin-1-yl)-3-(isopropylamino)propan-1-on oder ein pharmazeutisch annehmbares Salz davon ist, wobei die Mutation L52, E17 oder Q79 entspricht oder daran angeglichen ist.

2. Verfahren nach Anspruch 1, wobei die Zelle eine erhöhte Empfindlichkeit auf den AKT-Inhibitor aufweist.

3. Verfahren nach Anspruch 1 oder 2, wobei der AKT AKT1, AKT2 oder AKT3 ist.

4. Verfahren nach Anspruch 3, wobei AKT AKT1 ist.

5. Verfahren nach einem der Ansprüche 1 bis 4, wobei die Mutation E17 oder Q79 entspricht oder daran angeglichen ist.

6. Verfahren nach einem der Ansprüche 1 bis 4, wobei:
a. die L52-Mutation L52R entspricht oder daran angeglichen ist; und/oder
b. die E17-Mutation E17K entspricht oder daran angeglichen ist; und/oder
c. die Q79-Mutation Q79E entspricht oder daran angeglichen ist.

7. Verfahren nach Anspruch 4, umfassend das Bestimmen der Gegenwart einer Mutation von AKT1, wobei:
a. die Mutation L52R entspricht oder daran angeglichen ist; oder
b. die Mutation E17K entspricht oder daran angeglichen ist; oder
c. die Mutation Q79E entspricht oder daran angeglichen ist.

8. Verfahren nach einem der Ansprüche 1 bis 7, wobei die Mutation von AKT: (i) die Wechselwirkung zwischen der PH-Domäne und der Kinasedomäne von AKT unterbricht; oder (ii) die Phosphorylierung von AKT verstärkt und gegebenenfalls wobei die Verstärkung der Phosphorylierung aufgrund einer konstitutiven Phosphorylierung stattfindet.

9. Verfahren nach einem der Ansprüche 1 bis 8, wobei das Verfahren weiters das Bestimmen des PTEN-Status der Zelle umfasst, wobei ein PTEN-Verlust mit erhöhter Empfindlichkeit auf den Inhibitor korreliert.

10. Verfahren nach einem der Ansprüche 1 bis 9, wobei die Tumorzelle eine Prostatakrebszelle, eine Eierstockkrebszelle, eine Brustkrebszelle, eine Magenkrebszelle oder eine Pankreaskrebszelle ist; oder wobei die Tumorzelle eine endokrine resistente Brustkrebszelle ist.

11. Verfahren nach einem der Ansprüche 1 bis 10, wobei das Verfahren ferner das Bestimmen der Gegenwart einer PI3K-Mutation der Zelle umfasst, wobei die Gegenwart einer PI3K-Mutation mit einer erhöhten Empfindlichkeit gegenüber einer Inhibierung durch den Inhibitor korreliert, gegebenenfalls wobei die PI3K-Mutationen eine oder beide aus H1047R und H1047L sind.

12. Verfahren nach einem der Ansprüche 1 bis 11, wobei das Verfahren weiters das Bestimmen des pAKT-Profils der Zelle umfasst, wobei: (i) ein hohes Aktivitätsprofil für pAKT mit einer erhöhten Empfindlichkeit gegenüber einer Inhibierung durch den Inhibitor korreliert; oder (ii) ein niedriges Aktivitätsprofil für pAKT mit einer verringerten Empfindlichkeit gegenüber einer Inhibierung durch den Inhibitor korreliert.

13. Verfahren nach einem der Ansprüche 1 bis 12, wobei das Verfahren weiters Folgendes umfasst:
(i) Bestimmen des Her2- und/oder Her3-Status der Zelle, wobei ein Her2+-Status und/oder ein Her3+-Status der Zelle mit erhöhter Empfindlichkeit gegenüber einer Inhibierung durch den Inhibitor korreliert; oder
(ii) Bestimmen der Gegenwart einer Met-Kinase-Mutation der Zelle, wobei die Gegenwart einer Met-Kinase-Mutation mit der Empfindlichkeit der Zelle auf den AKT-Inhibitor korreliert; oder
(iii) Bestimmen der Gegenwart einer B-Raf- oder K-Ras-Mutation der Zelle, wobei die Gegenwart einer B-Raf- oder K-Ras-Mutation mit einer Verringerung der Empfindlichkeit der Zelle auf den AKT-Inhibitor korreliert; oder
(iv) Bestimmen des Lokalisierungsprofils von FOXO3a in der Zelle, wobei ein zytoplasmatisches Lokalisierungsprofil von FOXO3a mit der Empfindlichkeit der Zelle gegenüber einer Inhibierung durch den Akt-Inhibitor korreliert; oder
(v) Bestimmen des INPP4B-, PHLPP- und/oder PP2A-Status der Zelle, wobei ein Verlust an INPP4B, PHLPP und/oder PP2A mit einer erhöhten Empfindlichkeit auf den Inhibitor korreliert.

14. Verfahren nach einem der Ansprüche 1 bis 13, das weiters das Bestimmen der Gegenwart der Mutation von AKT in einer physiologischen Probe von einem Patienten umfasst und weiters gegebenenfalls das Informieren eines Patienten, für den die Gegenwart einer Mutation von AKT bestimmt wird, umfasst, dass ein AKT-Inhibitor verabreicht oder nicht verabreicht werden sollte.

15. (S)-2-(4-Chlorphenyl)-1-(4-((5R,7R)-7-hydroxy-5-methyl-6,7-dihydro-5H-cyclopenta-[d]pyrimidin-4-yl)piperazin-1-yl)-3-(isopropylamino)propan-1-on oder ein pharmazeutisch annehmbares Salz davon zur Verwendung in einem Verfahren zur Behandlung einer Krebszelle, umfassend eine oder mehrere AKT-Mutationen, wobei die Mutation L52, E17 oder Q79 entspricht oder daran angeglichen ist.

## Revendications

1. Procédé de prédiction de la sensibilité de la croissance d'une cellule tumorale à un inhibiteur d'AKT, comprenant la détermination de la présence d'une mutation à AKT, dans lequel la présence d'une mutation en AKT est corrélée à la sensibilité de la cellule à l'inhibiteur d'AKT, dans lequel l'inhibiteur d'AKT est la (S)-2-(4-chlorophényl)-1-(445R,7R)-7-hydroxy-5-méthyl-6,7-dihydro-5H-cyclopenta[d]pyrimidin-4-yl)pipérazin-1-yl)-3-(isopropylamino)propan-1-one, ou un sel pharmaceutiquement acceptable de celle-ci, dans lequel la mutation correspond à, ou s'aligne avec, L52, E17 ou Q79.

2. Procédé selon la revendication 1, dans lequel la cellule présente une sensibilité accrue à l'inhibiteur d'AKT.

3. Procédé selon la revendication 1 ou 2, dans lequel l'AKT est AKT1, AKT2 ou AKT3.

4. Procédé selon la revendication 3, dans lequel l'AKT est AKT1.

5. Procédé selon l'une quelconque des revendications 1 à 4, dans lequel la mutation correspond à, ou s'aligne avec, E17 ou Q79.

6. Procédé selon l'une quelconque des revendications 1 à 4, dans lequel :
a. la mutation L52 correspond à, ou s'aligne avec, L52R ; et/ou
b. la mutation E17 correspond à, ou s'aligne avec, El7K ; et/ou
c. la mutation Q79 correspond à, ou s'aligne avec, Q79E.

7. Procédé selon la revendication 4, comprenant la détermination de la présence d'une mutation en AKT1, dans lequel :
a. la mutation correspond à, ou s'aligne avec, L52R ; ou
b. la mutation correspond à, ou s'aligne avec, El7K ; ou
c. la mutation correspond à, ou s'aligne avec, Q79E.

8. Procédé selon l'une quelconque des revendications 1 à 7, dans lequel la mutation en AKT : (i) perturbe l'interaction entre le domaine PH et le domaine kinase d'AKT ; ou (ii) augmente la phosphorylation d'AKT, et facultativement dans lequel l'augmentation de la phosphorylation est due à une phosphorylation constitutive.

9. Procédé selon l'une quelconque des revendications 1 à 8, dans lequel le procédé comprend en outre la détermination du statut PTEN de la cellule, dans lequel la perte de PTEN est corrélée à une sensibilité accrue à l'inhibiteur.

10. Procédé selon l'une quelconque des revendications 1 à 9, dans lequel la cellule tumorale est une cellule cancéreuse de la prostate, une cellule cancéreuse de l'ovaire, une cellule cancéreuse du sein, une cellule cancéreuse gastrique ou une cellule cancéreuse du pancréas ; ou dans lequel la cellule tumorale est une cellule cancéreuse du sein résistant à l'endocrine.

11. Procédé selon l'une quelconque des revendications 1 à 10, dans lequel le procédé comprend en outre la détermination de la présence d'une mutation PI3K de la cellule, dans lequel la présence d'une mutation PI3K est corrélée avec une sensibilité accrue à l'inhibition par l'inhibiteur, facultativement dans lequel lesdites mutations PI3K sont l'une ou les deux parmi H1047R et H1047L.

12. Procédé selon l'une quelconque des revendications 1 à 11, dans lequel le procédé comprend en outre la détermination du profil pAKT de la cellule, dans lequel : (i) un profil d'activité élevé pour les pAKT est corrélé avec une sensibilité accrue à l'inhibition par l'inhibiteur ; ou (ii) un profil d'activité faible pour pAKT est corrélé avec une sensibilité réduite à l'inhibition par l'inhibiteur.

13. Procédé selon l'une quelconque des revendications 1 à 12, dans lequel le procédé comprend en outre les étapes consistant à : (i) déterminer le statut Her2 et/ou Her3 de la cellule, dans lequel un statut Her2+ et/ou Her3+ de la cellule est corrélé avec une sensibilité accrue à l'inhibition par l'inhibiteur ; ou
(ii) déterminer la présence d'une mutation de kinase Met de la cellule, dans lequel la présence d'une mutation de kinase Met est corrélée avec la sensibilité de la cellule à l'inhibiteur d'AKT ; ou
(iii) déterminer la présence d'une mutation B-Raf ou K-Ras de la cellule, dans lequel la présence d'une mutation B-Raf ou K-Ras est corrélée avec une diminution de la sensibilité de la cellule à l'inhibiteur d'AKT ; ou
(iv) déterminer le profil de localisation de FOXO3a dans la cellule, dans lequel un profil de localisation cytoplasmique de FOXO3a est en corrélation avec la sensibilité de la cellule à l'inhibition par l'inhibiteur d'Akt ; ou
(v) déterminer le statut INPP4B, PHLPP et/ou PP2A de la cellule, dans lequel la perte INPP4B, PHLPP et/ou PP2A est corrélée à une sensibilité accrue à l'inhibiteur.

14. Procédé selon l'une quelconque des revendications 1 à 13, comprenant en outre la détermination de la présence de la mutation en AKT dans un échantillon physiologique provenant d'un patient, et comprenant en outre facultativement l'étape consistant à informer un patient, pour lequel la présence d'une mutation en AKT est déterminée, qu'un inhibiteur d'AKT doit être administré ou ne doit pas être administré.

15. (S)-2-(4-chlorophényl)-1-(4-((5R,7R)-7-hydroxy-5-méthyl-6,7-dihydro-5H-cyclopenta[d]pyrimidin-4-yl)pipérazin-1-yl)-3-(isopropylamino)propan-1-one, ou un sel pharmaceutiquement acceptable de celle-ci, à utiliser dans un procédé de traitement d'une cellule cancéreuse comprenant une ou plusieurs mutations en AKT, dans laquelle la mutation correspond à, ou s'aligne avec, L52, E17 ou Q79.
